**Europäisches Patentamt**

**European Patent Office**

**Office européen des brevets**

(19)

(11) Veröffentlichungsnummer: **0 154 940**
**B1**

(12) # EUROPÄISCHE PATENTSCHRIFT

(45) Veröffentlichungstag der Patentschrift: **05.09.90**

(21) Anmeldenummer: **85102588.2**

(22) Anmeldetag: **07.03.85**

(51) Int. Cl.⁵: **A 61 K 37/43**

(54) **Neue Verwendung von Somatostatin.**

(30) Priorität: **13.03.84 DE 3409062**
**30.10.84 DE 3439716**

(43) Veröffentlichungstag der Anmeldung:
**18.09.85 Patentblatt 85/38**

(45) Bekanntmachung des Hinweises auf die
Patenterteilung:
**05.09.90 Patentblatt 90/36**

(84) Benannte Vertragsstaaten:
**BE CH DE FR GB IT LI LU NL SE**

(56) Entgegenhaltungen:
**DE-A-3 120 929**
**DE-A-3 416 374**
**US-A-4 366 148**

(73) Patentinhaber: **CuraMED Pharma GmbH**
**Oltmannsstrasse 11**
**D-7800 Freiburg (DE)**

(72) Erfinder: **Chrubasik, Joachim, Dr. med.**
**Schöneckstrasse 13**
**D-7800 Freiburg (DE)**
Erfinder: **Chrubasik, geb. Hausmann, Sigrun, Dr.**
**med.**
**Schöneckstrasse 13**
**D-7800 Freiburg (DE)**

(74) Vertreter: **Werner, Hans-Karsten, Dr. et al**
**Deichmannhaus am Hauptbahnhof**
**D-5000 Köln 1 (DE)**

Courier Press, Leamington Spa, England.

EP 0 154 940 B1

(56) References cited:

CHEMICAL ABSTRACTS, Band 95, Nr. 21, 23. November 1981, Seite 47, Zusammenfassung Nr. 180827t, Columbus, Ohio, US; A. CHANDRA et al.: "Effects of intraventricular administration of neurotensin and somatostatin on thermoregulation in the rat", & NEUROPHARMACOLOGY 1981, 20(7), 715-18

CHEMICAL ABSTRACTS, Band 94, Nr. 15, 13. April 1981, Seite 89, Zusammenfassung Nr. 114853b, Columbus, Ohio, US; M.D. LUMPKIN et al.: "Paradoxical elevation of growth hormone by intraventricular somatostatin: possible ultra-short loop feedback", & SCIENCE (WASHINGTON, D.C., 1883-) 1981, 211(4486), 1072-4

CHEMICAL ABSTRACTS, Band 90, Nr. 13, 26. März 1979, Seite 74, Zusammenfassung Nr. 97875h, Columbus, Ohio, US; D. MALTHE-SORENSSEN et al.: "Modulation of the turnover rate of acetylcholine in rat brain by intraventricular injections of thyrotropin-releasing hormone, somatostatin, neurotensin and angiotensin II.", & J. NEUROCHEM. 1978, 31(3), 685-91

## Beschreibung

Gegenstand der vorliegenden Erfindung ist eine neue Verwendung von Somatostatin.

Somatostatin ist ein synthetisches Peptid, das bisher gegen schwere akute gastroduodenale Ulkusblutungen, schwere akute Blutungen bei akuter erosiver bzw. hämorrhagischer Gastritis und Prophylaxe von postoperativen pankreatischen Komplikationen nach Pankreaschirurgie eingesetzt wurde. Somatostatin hemmt die Freisetzung von Wachstumshormon sowie anderen Hormonen, wie TSH, Insulin, Glucagon, Gastrin, Sekretin und Cholezystokinin. Weiterhin wurde von M. Rezek et al., Can.J.Physiol.Pharmakol. 56, 227-231, 1977 beschrieben, daß nach intrazerebroventrikulärer Infusion bei Ratten eine kurzfristige analgetische Wirkung gefunden wurde. Die Halbwertzeit von Somatostatin ist mit 2 — 4 Minuten außerordentlich kurz, jedoch ist in der Humanmedizin eine intrazerebroventrikuläre Dauerinfusion nicht durchführbar.

Die ältere, nicht vorveröffentlichte DE-OS 34 16 374 beschreibt die Verwendung von Somatostatin zur Behandlung idiopathischer Kopfschmerzen.

Es wurde jetzt überraschenderweise gefunden, daß Somatostatin nach periduraler Applikation rasch und in hohem Maße die Dura zu penetrieren vermag und in den Liquor gelangt. Damit kann Somatostatin zu den Opiatrezeptoren am Rückenmark gelangen und dort seine überraschend gute und bisher unbekannte analgetische Wirkung entwickeln. Weiterhin wurde gefunden, daß auch intraspinale und sogar intraventrikuläre Injektionen und Infusionen von Somatostatin beim Menschen zu einer ausgeprägten, dem Morphin vergleichbaren, analgetischen Wirkung führen. Dazu wurden Krebspatienten mit unerträglichen Schmerzen im terminalen Stadium oder auch bei postoperativen Schmerzen 250 µg Somatostatin als Injektion und danach 10 bis 50 µg Somatostatin pro Stunde als Infusion teils intraspinal, teils intraventrikulär appliziert. Diese Behandlungen führten zu einer ausgeprägten Analgesie, die der des Morphins vergleichbar ist. Die Infusionen wurden länger als 2 Wochen nebenwirkungsfrei vertragen.

Es wurde weiterhin gefunden, daß intradurale oder intraspinale Injektionen und Infusionen anästhetische Wirkung haben und anstelle üblicher Anästhetika eingesetzt werden können.

Damit öffnen sich für Somatostatin völlig neue und hoch interessante Anwendungsgebiete, zumal die sonstigen Wirkungen und Nebenwirkungen von Somatostatin gut untersucht und bekannt sind.

Gegenstand der vorliegenden Erfindung ist somit die Verwendung von Somatostatin zur Herstellung von periduralen, intraspinalen oder intraventrikulären Injektionslösungen als Analgetikum oder Anästhetikum.

Als peridurale, intraspinale oder intraventrikuläre Injektionslösungen können prinzipiell auch die bisher bekannten Lösungen zur Injektion und zur Infusion verwendet werden, die in isotonischer, steriler, pyrogenfreier Natriumchloridlösung vorliegen.

Wegen der kurzen Halbwertzeit des Peptides muß die Applikationsform angepaßt werden. Dazu eignen sich beispielsweise kleine, extern anbringbare Dosiergeräte, die eine Dauerinfusion gestatten. Bei der intraventrikulären Applikation wird durch eine künstliche Öffnung des Kopfes Zugang zum Ventrikel geschaffen und hierhinein die Infusionslösung geleitet.

Bei der Untersuchung von Hunden wurde festgestellt, daß die analgetische Wirksamkeit mehrere Wochen lang anhält. Die Analgesieprüfung nach Cohen (J.Surg. 32, 32-37, 1982) ergab, daß alle Tiere eine 60°C Wärmeplatte über die Dauer von 5 Sekunden tolerierten. Ein Hund mit einem großen Dekubitus zeigte kein Zeichen von Schmerz. Die Hunde waren jedoch nicht sediert und zeigten eine ausgeglichene Verhaltensweise.

Die Versuche an den Hunden verliefen insgesamt nebenwirkungsfrei.

Erste Humanversuche haben bestätigt, daß die peridurale Applikation von 125 µg bzw. 250 µg Somatostatin analgetisch wirksam sind.

Trotz der guten Verträglichkeit wird man die jeweils applizierte Menge an Somatostatin so niedrig wie möglich halten. Einige Ergebnisse deuten darauf hin, daß die Dosierung niedriger als bisher gewählt werden kann.

### Patentanspruch

Verwendung von Somatostatin zur Herstellung von periduralen, intraspinalen oder intraventrikulären Injektions- und Infusionslösungen als Analgetikum oder Anästhetikum.

### Revendication

Utilisation de la somatostatine pour l'obtention de solutions pour injection ou perfusion péridurale, intraspinale ou intraventriculaire, utilisables comme analgésique ou anesthésiant.

### Claim

Use of somatostatin as analgetic or anaesthetic drug for the manufacture of peridural, intraspinal or intraventricular solutions for injections and infusions.